# EUROPÄISCHE PATENTSCHRIFT

(11) **EP 2 124 924 B1**
(45) Veröffentlichungstag und Bekanntmachung des Hinweises auf die Patenterteilung: **27.06.2012**
(21) Anmeldenummer: 07856866.4
(22) Anmeldetag: 19.12.2007
(51) Int. Cl.: A61K 31/203, A61K 31/232, A61K 31/565, A61K 31/566, A61K 31/567, A61K 31/569, A61K 31/57, A61K 31/575, A61K 31/585, A61P 15/18, A61P 17/00, A61P 17/06, A61P 17/08, A61P 17/10

(54) **Wirkstoffkombination aus einem Retinoid und einer kontrazeptiv wirkenden Hormonkombination als Arzneimittel zur Behandlung von Hauterkrankungen**
Active ingredient combination of a retinoid and a hormone combination with contraceptive action as medicament for treatment of skin diseases
Combinaison d'agents actifs composée d'un rétinoïde et d'une combinaison d'hormones à effet contraceptif servant de médicament pour le traitement de maladies cutanées

(30) Priorität: 22.12.2006 DE 102006062119
(43) Veröffentlichungstag der Anmeldung: 02.12.2009
(73) Patentinhaber: Grünenthal GmbH, 52078 Aachen (DE)
(72) Erfinder: SCHRAMM, Georg, 52224 Stolberg (DE); OEDEKOVEN, Karl-Heinz, 52223 Stolberg (DE)
(74) Vertreter: Kutzenberger, Helga
(86) Internationale Anmeldenummer: PCT/EP2007/011143
(87) Internationale Veröffentlichungsnummer: WO 2008/077541

(56) Entgegenhaltungen:
- WO-A-2005/115402
- US-A1- 2007 254 025
- SHETH REKHA ET AL: "Isotretinoin: an Indian experience" INDIAN JOURNAL OF DERMATOLOGY, VENEREOLOGY AND LEPROLOGY, DEPARTMENT OF DERMATOLOGY & VENEREOLOGY,, IN, Bd. 67, Nr. 4, Juli 2001 (2001-07), Seiten 180-182, XP008090029
- ZOUBOULIS C C ET AL: "UPDATE AND FUTURE OF SYSTEMIC ACNE TREATMENT" DERMATOLOGY, Bd. 206, Nr. 1, 2003, Seiten 37-53, XP008021864 ISSN: 1018-8665
- HONMA S ET AL: "IDENTIFICATION AND ANTI-ANDROGENIC ACTIVITY OF THE METABOLITES OF 17ALPHA-ACETOXY-6-CHLOROPREGNA-4,6-DIENE-3 ,20-DIONE (CHLORMADINONE ACETATE) IN THE RAT, RABBIT, DOG AN DMAN" CHEMICAL AND PHARMACEUTICAL BULLETIN, PHARMACEUTICAL SOCIETY OF JAPAN, TOKYO, JP, Bd. 25, Nr. 8, 25. August 1977 (1977-08-25), Seiten 2019-2031, XP009083454 ISSN: 0009-2363
- THIELITZ ANJA ET AL: "[Systemic acne therapy]" JOURNAL DER DEUTSCHEN DERMATOLOGISCHEN GESELLSCHAFT = JOURNAL OF THE GERMAN SOCIETY OF DERMATOLOGY : JDDG MAY 2005, Bd. 3, Nr. 5, Mai 2005 (2005-05), Seiten 366-378 ; qui, XP002475490 ISSN: 1610-0379
- KATSAMBAS A ET AL: "Acne: Systemic treatment" CLINICS IN DERMATOLOGY, J.B. LIPPINCOTT, PHILADELPHIA, PA, US, Bd. 22, Nr. 5, September 2004 (2004-09), Seiten 412-418, XP004647113 ISSN: 0738-081X

## Beschreibung

Die vorliegende Erfindung betrifft ein Arzneimittel, dessen Wirkstoffkombination aus einem Retinoid ausgewählt aus der Gruppe bestehend aus Acitretin [9-(4-Methoxy-2,3,6-trimethyl-phenyl)-3,7-dimethyl-nona-2,4,6,8-tetraensäure], Etretinat [Ethyl 9-(4-methoxy-2,3,6-trimethyl-phenyl)-3,7-dimethyl-nona-2,4,6,8-tetraenat], Isotretinoin [3,7-Dimethyl-9-(2,6,6-trimethyl-1-cyclo-hexen-1-yl)-2,4,6,8-nonatetraensäure] und Tretinoin [3,7-Dimethyl-9-(2,6,6-trimethyl- 1-cyclohexenyl)nona-2,4,6,8-tetraensäure] und aus einer kontrazeptiv wirkenden Hormonkombination aus einer Östrogenkomponente und einer Gestagenkomponente besteht, sowie dessen Einsatz als Darreichungsform zur Behandlung Akne, Seborrhöe oder Psoriasis.

Akne ist eine entzündliche Erkrankung der Talgdrüsen, die vor allem in der Pubertät auftritt. In ihrer leichten Erscheinungsform stellt Akne eine mehr oder weniger oberflächliche Beeinträchtigung dar, die sich in leichten fleckigen Reizungen zeigt und mit Dermato-Kosmetika ausreichend behandelt werden kann. Bei den entzündlichen Erscheinungsformen der Akne dringen jedoch Bakterien in oder um die Haarfollikel ein, wodurch es zur Bildung von Papeln, Pusteln, Knoten, infizierten Taschen und im Extremfall zu infizierten Zysten kommt. Diese Entzündungen können sich stark ausdehnen und dauerhafte Narben bilden.

Mindestens 80% der Teenager sind von Akne betroffen. Durch Akne hervorgerufene Gesichtsausschläge können gerade in der Pubertät ein Problem darstellen, da sie das äußere Erscheinungsbild der heranwachsenden Person beeinträchtigen und in vielen Fällen psychische Störungen, insbesondere bei Mädchen, verursachen können. Eine therapeutische Behandlung von Akne ist daher von außerordentlicher Wichtigkeit.

Retinoide sind seit langem bekannt. Sie werden als pharmazeutische Wirkstoffe in Medikamenten zur Behandlung von Hautkrankheiten wie beispielsweise Psoriasis oder Akne, insbesondere von schwerer Akne, verwendet (vgl. z.B. Mezick et al., J. Invest. Dermatol., 83:110-13, 1984). So wird das Retinoid Isotretinoin (13-*cis*-Retinsäure), ein *cis*-Isomer des Tretinoin, zur Herstellung eines hochwirksamen, aber auch nebenwirkungsreichen Aknemedikaments eingesetzt. Eine Therapie mit Isotretinoin bringt womöglich, wie dies bei den meisten oral zu verabreichenden Retinoiden der Fall ist, erhebliche Risiken mit sich, da dieser Wirkstoff eine Vielzahl von Nebenwirkungen aufweist. Wegen der Nebenwirkungen werden daher meist nur schwere und therapieresistente Krankheitsverläufe mit Isotretinoin behandelt.

Eine der schwerwiegendsten Nebenwirkungen ist eine starke Teratogenität (fruchtschädigende Wirkung) des Wirkstoffs, weshalb Retinoide bei Frauen im gebärfähigen Alter nur unter Ausschluss einer Schwangerschaft eingenommen werden dürfen. Aus diesem Grund werden die mit Retinoiden behandelten Patientinnen angehalten, eine Schwangerschaft während der Behandlungsdauer zu vermeiden, indem sie vorzugsweise Kontrazeptiva einnehmen.
R. Sheth und V. Poonevala beschreiben in "ISOTRETINOIN : AN INDIAN EXPERIENCE" (Indian J. Dermatol. Venereol. Leprol. 2001, 67, 180-182) die Behandlung von Akne, unter anderem Acne vulgaris, Rosazea und weiterer Hauterkrankungen mit Retinoiden, insbesondere Isotretinoin. 11 Frauen wurde dabei als Arzneimittel Isotretinoin zusammen mit Diane, d.h. mit einer Hormonkombination von Cyproteronacetat und Ethinylestradiol, verabreicht.

Aufgabe der vorliegenden Erfindung war es daher, bei einer Behandlung mit einem retinoidhaltigen Arzneimittel die notwendige Kontrazeption bestmöglichst zu garantieren.

Diese Aufgabe wird erfindungsgemäß durch das zur Verfügung stellen einer Darreichungsform eines Arzneimittels, dessen Wirkstoffkombination aus einem zur Aknebehandlung verwendeten Retinoid und aus einer kontrazeptiv wirkenden Hormonkombination aus einer Östrogenkomponente und einer Gestagenkomponente gemäß Anspruch 1 besteht, gelöst.

Gegenstand der vorliegenden Erfindung ist daher eine Darreichungsform, dessen Wirkstoffkombination aus einem Retinoid ausgewählt aus der Gruppe bestehend aus Acitretin [9-(4-Methoxy-2,3,6-trimethyl-phenyl)-3,7-dimethyl-nona-2,4,6,8-tetraensäure], Etretinat [Ethyl 9-(4-methoxy-2,3,6-trimethyl-phenyl)-3,7-dimethyl-nona-2,4,6,8-tetraenat], Isotretinoin [3,7-Dimethyl-9-(2,6,6-trimethyl-1-cyclo-hexen-1-yl)-2,4,6,8-nonatetraensäure] und Tretinoin [3,7-Dimethyl-9-(2,6,6-trimethyl- 1-cyclohexenyl)nona-2,4,6,8-tetraensäure] und aus einer kontrazeptiv wirkenden Hormonkombination aus einer nachstehend aufgeführten Östrogenkomponente und einer nachstehend aufgeführten Gestagenkomponente besteht.

Durch das erfindungsgemäße Arzneimittel wird eine wirksame Behandlung unter Minimieren eines Schwangerschaftsrisikos erzielt, da eine weit bessere Einhaltung der Therapievorschrift sichergestellt ist.

Retinoide sind chemische Substanzen, die in ihrer chemischen Struktur oder in ihrer biologischen Aktivität mit dem Retinol (Vitamin A) verwandt sind. Ausgewählte Reti-noide kommen medizinisch-therapeutisch beim Menschen zum Einsatz. Drei Generationen von therapeutisch wirksamen Retinoiden sind bekannt:
- Nicht-aromatische Retinoide (1. Generation). Zu den nicht-aromatischen Retinoide zählen Tretinoin (all-trans-Retinsäure) und sein Isomer Isotretinoin (13-cis-Retinsäure). Beide entstehen in vergleichsweise geringer Menge auch *in vivo* als Metaboliten des Vitamins A.
- Mono-aromatische Retinoide (2. Generation), z.B. Acitretin, Etretinat oder Motretinid,
- Poly-aromatische Retinoide (3. Generation), z.B. Adapalen, Arotinoid, Acetylenretinoide oder Tazaroten.

Hinsichtlich der Nomenklatur von Retinoiden wird auf die Veröffentlichung "Nomenclature of Retinoids" der International Union of Pure and Applied Chemistry (IUPAC), Pure Appl Chem, 1983, V55(4), p.721-726 verwiesen.

Therapeutisch wirksame Retinoide, die in der erfindungsgemäßen Wirkstoffkombination vorliegen, sind Retinoide der 1. Generation, 2. Generation oder der 3. Generation. Bevorzugt wird als Retinoid wenigstens ein Retinoid ausgewählt aus der Gruppe bestehend aus Acitretin [9-(4-Methoxy-2,3,6-trimethyl-phenyl)-3,7-dimethyl-nona-2,4,6,8-tetraensäure], Etretinat [Ethyl 9-(4-methoxy-2,3,6-trimethyl-phenyl)-3,7-dimethyl-nona-2,4,6,8-tetraenat], Isotretinoin [3,7-Dimethyl-9-(2,6,6-trimethyl-1-cyclohexen-1-yl)-2,4,6,8-nonatetraensäure] und Tretinoin [3,7-Dimethyl-9-(2,6,6-trimethyl-1-cyclohexenyl)nona-2,4,6,8-tetraensäure], besonders bevorzugt Isotretinoin [3,7-Dimethyl-9-(2,6,6-trimethyl-1-cyclo-hexen-1-yl)-2,4,6,8-nonatetraensäure] eingesetzt.

Als Östrogenkomponente für die zum Einsatz kommende Hormonkombination wird wenigstens eine Verbindung ausgewählt aus der Gruppe bestehend aus Ethinylestradiol oder Östradiol, ganz besonders bevorzugt Östradiol eingesetzt, da dieses Östrogen als natürlich vorkommendes Östrogen die Leber weniger belastet.

Die Gestagenkomponente der erfindungsgemäß zum Einsatz kommenden Hormonkombination ist wenigstens eine Verbindung ausgewählt aus der Gruppe bestehend aus Chlormadinonacetat, 3α-Hydroxy-Chlormadinonacetat und 3β-Hydroxy-Chlormadinonacetat.

Bevorzugt wird als Gestagenkomponente in dem erfindungsgemäßen Arzneimittel ein antiandrogenes Gestagen zur Behandlung von Akne, Seborrhöe oder Psoriasis eingesetzt.

Die Darreichungsform liegt in Form einer bestimmten Anzahl von Tageseinheiten, zur oralen Verabreichung vor. Vorzugsweise sind eine bestimmte Anzahl dieser die Wirkstoffkombination enthaltenden Tageseinheiten für eine ununterbrochene, tägliche orale Verabreichung in Kombination mit einer bestimmten Anzahl hormonfreier, nur das Retinoid als Wirkstoff enthaltenden Tageseinheiten für eine daran anschließende, ununterbrochene, tägliche, orale Verabreichung an Frauen zu einer erfindungsgemäßen Darreichungsform zusammengefasst.

Bevorzugt weist die erfindungsgemäße Darreichungsform wenigstens 21 - 25 die Wirkstoffkombination enthaltenden Tageseinheiten und 7 bis 3 hormonfreie, nur das Retinoid als Wirkstoff enthaltende Tageseinheiten auf.

So kann beispielsweise die maximale Anzahl der Tageseinheiten einer erfindungsgemäßen Darreichungsform für eine ununterbrochene, tägliche, orale Verabreichung über 364 Tage entsprechen, wobei 357 bis 361, die Wirkstoffkombination enthaltende Tageseinheiten zur ununterbrochenen, täglichen, oralen Verabreichung und 7 bis 3 hormonfreie, das Retinoid enthaltende Tageseinheiten zur anschließenden ununterbrochenen, täglichen, oralen Verabreichung an Frauen vorliegen.

Bevorzugte Ausführungen hinsichtlich der maximalen Anzahl der die erfindungsgemäße Wirkstoffkombination enthaltenden Tageseinheiten bzw. der hormonfreien Tageseinheiten der erfindungsgemäßen Darreichungsform für eine ununterbrochene Verabreichung sind in Tabelle 1 zusammengefasst.

**Tabelle 1**

| Dauer der ununterbrochenen Verabreichung (Tage) | Anzahl der die Wirkstoffkombination enthaltenden Tageseinheiten | Anzahl hormonfreier, nur Retinoid-haltigen Tageseinheiten |
|---|---|---|
| 364 | 357-351 | 7-3 |
| 336 | 329-333 | 7-3 |
| 308 | 301-305 | 7-3 |
| 280 | 273-277 | 7-3 |
| 252 | 245-249 | 7-3 |
| 224 | 217-221 | 7-3 |
| 196 | 189-193 | 7-3 |
| 168 | 161-165 | 7-3 |
| 140 | 133-137 | 7-3 |
| 112 | 105-109 | 7-3 |
| 84 | 77-81 | 7-3 |
| 56 | 49-53 | 7-3 |
| 28 | 21-25 | 7-3 |

Weiterhin ist eine Darreichungsform bevorzugt, deren maximale Anzahl an Tageseinheiten für eine ununterbrochene, tägliche, orale Verabreichung über 168 Tage geeignet ist, wobei 161 bis 165 die Wirkstoffkombination enthaltenden Tageseinheiten zur ununterbrochenen, täglichen, oralen Verabreichung und 7 bis 3 hormonfreie, das Retinoid enthaltende Tageseinheiten zur anschließenden ununterbrochenen, täglichen, oralen Verabreichung an Frauen vorliegen.

Ebenso bevorzugt ist eine Darreichungsform, deren maximale Anzahl an Tageseinheiten für eine ununterbrochene, tägliche, orale Verabreichung über 112 Tage geeignet ist, wobei 105 bis 109 die Wirkstoffkombination enthaltenden Tageseinheiten zur ununterbrochenen, täglichen, oralen Verabreichung und 7 bis 3 hormonfreie, das Retinoid enthaltende Tageseinheiten zur anschließenden ununterbrochenen, täglichen, oralen Verabreichung an Frauen vorliegen.

Ebenso bevorzugt ist eine Darreichungsform, deren maximale Anzahl an Tageseinheiten für eine ununterbrochene, tägliche, orale Verabreichung über 84 Tage geeignet ist, wobei 77 bis 81 die erfindungsgemäße Wirkstoffkombination enthaltenden Tageseinheiten zur ununterbrochenen, täglichen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen.

Eine bevorzugte Ausführungsform der erfindungsgemäßen Darreichungsform weist eine maximale Anzahl an Tageseinheiten für eine ununterbrochene, tägliche, orale Verabreichung für 28 Tage auf, wobei 21 bis 25 die erfindungsgemäße Wirkstoffkombination enthaltenden Tageseinheiten zur ununterbrochenen, täglichen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen.

In einer weiterhin bevorzugten Ausführungsform besteht die erfindungsgemäße Darreichungsform aus bis zu 13 Anordnungen von jeweils 28 Tageseinheiten zur ununterbrochenen, täglichen, oralen Verabreichung, wobei jeweils 21 bis 25 die Wirkstoffkombination enthaltenden Tageseinheiten zur ununterbrochenen, täglichen Verabreichung mit jeweils 7 bis 3 hormonfreien, nur das Retinoid als Wirkstoff enthaltenden Tageseinheiten zur anschließenden ununterbrochenen, täglichen, oralen Verabreichung an Frauen kombiniert sind.

Gemäß dieser bevorzugten Ausführungsform kann die erfindungsgemäße Darreichungsform auch aus bis zu 6, vorzugsweise bis zu 4 dieser Anordnungen aus jeweils 21 bis 25 die Wirkstoffkombination enthaltenden Tageseinheiten zur ununterbrochenen, täglichen oralen Verabreichung kombiniert mit jeweils 7 bis 3 hormonfreien, nur das Retinoid als Wirkstoff enthaltenden Tageseinheiten zur anschließenden ununterbrochenen, täglichen, oralen Verabreichung an Frauen bestehen.

Bevorzugt weist jede, die hormonhaltige Wirkstoffkombination enthaltenden Tageseinheiten jeweils dieselbe Menge der Gestagenkomponente und jeweils dieselbe Menge der Östrogenkomponente auf.

Jede die hormonhaltige Wirkstoffkombination enthaltende Tageseinheit der erfindungsgemäßen Darreichungsform enthält vorzugsweise die Östrogenkomponente jeweils in einer den weiblichen Menstruationszyklus stabilisierenden Menge und die Gestagenkomponente jeweils in einer kontrazeptiv wirkenden Menge. Stabilisierende Mengen der Östrogenkomponente und kontrazeptiv wirkende Mengen der Gestagenkomponente sind dem Fachmann bekannt und beispielhaft in Tabelle 2 und 3 zusammengefasst.

**Tabelle 2**

| Östrogenkomponente | bevorzugte Wirkstoffmenge pro hormonhaltiger Tageseinheit |
|---|---|
| Ethinylestradiol | 5 bis 50 µg |
| Östradiol | 0,5 bis 5 mg |

**Tabelle 3**

| Gestagenkomponente | bevorzugte Wirkstoffmenge pro hormonhaltiger Tageseinheit |
|---|---|
| Chlormadinonacetat | 0,5 bis 10 mg |
| 3α-Hydroxy-Chlormadinonacetat | 1 bis 20 mg |
| 3β-Hydroxy-Chlormadinonacetat | 1 bis 20 mg |

Jede Tageseinheit der erfindungsgemäßen Darreichungsform enthält vorzugsweise 1 bis 75 mg und besonders bevorzugt 3 bis 30 mg Retinoid, wobei es zu Beginn einer Aknetherapie notwendig sein kann, das Retinoid in einer höheren Wirkstoffdosierung einzusetzen als im weiteren Verlauf.

Bevorzugt enthält daher jede der ersten zur Verabreichung vorgesehenen 28 Tageseinheiten einer Darreichungsform jeweils mindestens 10 mg des Retinoids und die darüberhinausgehenden, weiteren Tageseinheiten der Darreichungsform vorzugsweise jeweils 1/2 bis 1/5 der Retinoidmenge der ersten zur Verabreichung vorgesehenen 28 Tageseinheiten.

Vorzugsweise weisen diese zum Beginn der Verabreichung vorgesehenen 28 Tageseinheiten jeweils dieselbe Menge des Retinoids und die restlichen Tageseinheiten der Darreichungsform ebenfalls jeweils dieselbe Menge des Retinoids auf.

Die erfindungsgemäße Darreichungsform eignet sich bevorzugt zur Prophylaxe und/oder zur Behandlung von Akne. Eine Therapierung mit der erfindungsgemäßen Darreichungsform hat u. a. den großen Vorteil, dass während der üblichen Einnahmepause von hormonellen Kontrazeptiva die Aknebehandlung nicht unterbrochen wird und dementsprechend keine entscheidenden Schwankungen des Retinoids im Blutspiegel der Patientin auftritt.

Die Akne ist hauptsächlich eine Erkrankung des Talgdrüsenfollikels, die zunächst nichtentzündliche Komedonen hervorbringt, im späteren Verlauf aber auch eine Reihe entzündlicher Effloreszenzen (u.a. Papeln, Pusteln und Knoten) entstehen lassen kann. Bei einigen Akneformen können Terminal- und Vellushaarfollikel betroffen sein.

Akne wird nach Ursache, Ausprägung bzw. Schweregrad und Lebensalter systematisiert.

Am bekanntesten und verbreitetsten ist die *Acne vulgaris* oder "gewöhnliche Akne". "Gewöhnlich" bedeutet in diesem Zusammenhang, dass die Akne, bedingt durch verstärkten Androgeneinfluss während der Pubertät auftritt und spätestens zum Anfang des 3. Lebensjahrzehnts abklingt. Weil ihre Ursachen in Veränderungen des Körperstoffwechsels liegen, gehört die *Acne vulgaris* zu den endogenen Akneformen.

Tritt die Akne mutmaßlich endogener Ursache in "ungewöhnlichem" Lebensalter auf, also entweder präpubertär oder im fortgeschrittenen Erwachsenenalter (*Acne tarda*), sollte nach dafür typischen Stoffwechselerkrankungen gesucht werden.

Die *Acne inversa* ist eine häufig schwere Entzündung der Talgdrüsen und Terminalhaarfollikel, vorzugsweise in intertriginösen Arealen wie z.B. Achselhöhle, Leistenregion und Gesäßfalte.

Akne kann isoliert, aber auch in Kombination mit anderen Hauterkrankungen, wie beispielsweise Psoriasis, Seborrhöe und/oder oder Rosacea auftreten, bzw. eine Erkrankung kann die andere auslösen und/oder bedingen.

Seborrhöe ist ein medizinischer Begriff für die krankhaft veränderte Absonderung der Talgdrüsen.

Er wird primär mit Androgenen, höherer Androgenrezeptordichte, Androgenrezeptorempfindlichkeit, mit Mangel an Biotin (Vitamin B7) sowie Vitamin B6 (Pyridoxin) in Verbindung gebracht. Der Begriff wird manchmal auch für das Seborrhoische Ekzem benutzt. *Seborrhoe oleosa* bewirkt eine ölige Beschaffenheit der Haut, *Seborrhoe sicca* manifestiert sich in kleieförmigen, fettigen Schuppungen. Die Seborrhöe fördert unter anderem das Auftreten von *Rosacea, Acne vulgaris* und *Acne necroticans.*

Die Schuppenflechte oder die **Psoriasis** ist eine Hautkrankheit, die sich nach außen hin im wesentlichen durch einige stark schuppende, punktförmige bis handtellergroße Hautstellen (häufig an den Knien, Ellenbogen und der Kopfhaut) zeigt. Die Schuppenflechte ist eine nicht-ansteckende Autoimmunkrankheit und kann neben der Haut auch Gelenke (5 - 20 %) und Finger-/Zehennägel befallen.

Die häufigst vorkommende Form der Psoriasis ist die *Psoriasis vulgaris.*

Ein weiterer Aspekt der vorliegenden Erfindung betrifft die Verwendung wenigstens eines Retinoid und einer kontrazeptiv wirkenden Hormonkombination aus einer Östrogenkomponente und einer Gestagenkomponente gemäß Anspruch 1 zur Herstellung einer Darreichungsform in Form der vorstehend beschriebenen Tageseinheiten A und hormonfreien Tageseinheiten B zur Prophylaxe und/oder zur Behandlung von Akne, wobei die Akne vorzugsweise ausgewählt ist aus der Gruppe, umfassend *Acne aestivalis, Acne aggregata, Acne comedonica, Acne conglobata, Acne inversa, inversa, Acne nodularis, Acne papulopustulosa* und *Acne vulgaris.*

Eine bevorzugte Ausführungsform der Erfindung betrifft die Verwendung eines Retinoids ausgewählt aus der Gruppe bestehend aus Acitretin [9-(4-Methoxy-2,3,6-trimethyl-phenyl)-3,7-dimethyl-nona-2,4,6,8-tetraensäure], Etretinat [Ethyl 9-(4-methoxy-2,3,6-trimethyl-phenyl)-3,7-dimethyl-nona-2,4,6,8-tetraenat], Isotretinoin [3,7-Dimethyl-9-(2,6,6-trimethyl-1-cyclo-hexen-1-yl)-2,4,6,8-nonatetraensäure] und Tretinoin [3,7-Dimethyl-9-(2,6,6-trimethyl- 1-cyclohexenyl)nona-2,4,6,8-tetraensäure], einer Östrogenkomponente ausgewählt aus der Gruppe bestehend aus Östradiol und Ethinylestradiol und einer Gestagenkomponente ausgewählt aus der Gruppe bestehend aus Chlormadinonacetat, 3β-Hydroxy-Chlormadinonacetat (17α-acetoxy-chloropregna-4,6-dien-3β-ol-20-on) und 3α-Hydroxy-Chlormadinonacetat (17α-acetoxy-chloropregna-4,6-dien-3α-ol-20-on) zur Herstellung einer Darreichungsform in Form der vorstehend beschriebenen Tageseinheiten A und hormonfreien Tageseinheiten B zur Prophylaxe und/oder zur Behandlung von Akne.

Ganz besonders bevorzugt ist die Verwendung von Isotretinoin [3,7-Dimethyl-9-(2,6,6-trimethyl-1-cyclo-hexen-1-yl)-2,4,6,8-nonatetraensäure], einer Östrogenkomponente ausgewählt aus der Gruppe bestehend aus Östradiol und Ethinylestradiol und einer Gestagenkomponente ausgewählt aus der Gruppe bestehend aus Chlormadinonacetat, 3β-Hydroxy-Chlormadinonacetat (17α-acetoxychloropregna-4,6-dien-3β-ol-20-on) und 3α-Hydroxy-Chlormadinonacetat (17α-acetoxy-chloropregna-4,6-dien-3α-ol-20-on) zur Herstellung einer Darreichungsform in Form der vorstehend beschriebenen Tageseinheiten A und hormonfreien Tageseinheiten B zur Prophylaxe und/oder zur Behandlung von Akne, Seborrhöe, Psoriasis und/oder Rosazea, besonders bevorzugt zur Behandlung von Akne: Die Darreichungsform weist vorzugsweise die Tageseinheiten in Form von Tabletten auf, die neben dem vorstehend angegebenen Wirkstoff bzw. die vorstehend angegebene Wirkstoffkombination vorzugsweise die üblichen Hilfsstoffe aufweisen.

Die nachfolgenden Beispiele dienen zur näheren Erläuterung der Erfindung :

### Beispiel 1:

### Zusammensetzung

| | Pro Tablette |
|---|---|
| Isotretinoin | 20 mg |
| Ethinylestradiol | 0,020 mg |
| Chlormadinonacetat | 2,000 mg |
| Povidon K30 | 3,000 mg |
| Lactose | 31,980 mg |
| Maisstärke | 12,000 mg |
| Magnesiumstearat | 0,500 mg |
| Hochdisperses Siliciumdioxid | 0,500 mg |

Isotretinoin (ISO), Ethinylestradiol (EE) und Povidone K 30 (Polyvinylpyrrolidon) wird in 600 ml Ethanol gelöst. Chlormadinonacetat (Partikelgröße 90% <50µm), Lactose und Maisstärke wird in einem Mischer/Granulierer (Diosna P25) 5 min gemischt und anschließend mit der ethanolischen ISO/EE/PVP Lösung durchfeuchtet und gemischt. Die feuchte Masse wird durch ein 3 mm Sieb getrieben und in einem Vakuumtrockenschrank getrocknet. Das trockene Granulat wird durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 70 mg gepresst. Die hormonfreien Tabletten weisen 34 mg Lactose auf.

Die Tabletten werden mit einem Lack auf Basis Methylhydroxypropylcellulose überzogen (z. B. Opadry YS-1-2184 vom Hersteller Colorcon); Überzugsmasse 2 mg pro Tablette. Die Darreichungsform umfasst 364 Tageseinheiten, von denen die letzten 7 hormonfrei sind, zur ununterbrochenen Verabreichung.

### Beispiel 2:

### Zusammensetzung

| | Pro Tablette |
|---|---|
| Isotretinoin | 20 mg |
| Östradiol | 1 mg |
| Chlormadinonacetat | 2,000 mg |
| Povidon K30 | 3,000 mg |
| Lactose | 31 mg |
| Maisstärke | 12,000 mg |
| Magnesiumstearat | 0,500 mg |
| Hochdisperses Siliciumdioxid | 0,500 mg |

Isotretinoin (ISO), Östradiol (ÖS) und Povidone K 30 (Polyvinylpyrrolidon) werden in 600 ml Ethanol gelöst. Chlormadinonacetat (Partikelgröße 90% <50µm), Lactose und Maisstärke werden in einem Mischer/Granulierer (Diosna P25) 5 min gemischt und anschließend mit der ethanolischen ISO/ÖS/PVP Lösung durchfeuchtet und gemischt. Die feuchte Masse wird durch ein 3 mm Sieb getrieben und in einem Vakuumtrockenschrank getrocknet. Das trockene Granulat wird durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 70 mg gepresst. Die hormonfreien Tabletten enthalten 34 mg Lactose.

Die Tabletten werden mit einem Lack auf Basis Methylhydroxypropylcellulose überzogen (z. B. Opadry YS-1-2184 vom Hersteller Colorcon); Überzugsmasse 2 mg pro Tablette. Die Darreichungsform besteht aus 364 Tageseinheiten, von denen die letzten 7 hormonfrei sind, zur ununterbrochenen Verabreichung.

### Beispiel 3:

### Zusammensetzung

| | Pro Tablette |
|---|---|
| Isotretinoin | 40 mg |
| Ethinylestradiol | 0,015 mg |
| Chlormadinonacetat | 2,000 mg |
| Povidon K30 | 4,000 mg |
| Lactose | 43,485 mg |
| Maisstärke | 10,000 mg |
| $Magnesiumstearat | 0,500 mg |

Isotretinoin (ISO), Ethinylestradiol (EE) und Povidone K 30 (PVP) werden in 950 ml Ethanol gelöst. Chlormadinonacetat (Partikelgröße 90% <50µm), Lactose und Maisstärke werden in einem Mischer/Granulierer (Diosna P25) 5 min gemischt und anschließend mit der ethanolischen ISO/EE/PVP Lösung durchfeuchtet und gemischt. Die feuchte Masse wird durch ein 3 mm Sieb getrieben und in einem Vakuumtrockenschrank getrocknet. Das trockene Granulat wird durch ein 0,6 mm Sieb desagglomeriert, mit Magnesiumstearat gemischt und auf einer Tablettenpresse mit 6 mm Stempeln zu Tabletten mit einem Gewicht von 100 mg gepresst. Die hormonfreien Tabletten enthalten 45,5 mg Lactose.

Die Tabletten werden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa · s, | 0,1351 kg |
| Polyethylenglykol 6000 | 0,0395 kg |
| Propylenglykol | 0,0054 kg |
| Gereinigtes Wasser | 1,6200 kg |

Die Darreichungsform enthält mit 196 Tageseinheiten, von denen die letzten 7 hormonfrei sind, zur ununterbrochenen Verabreichung.

## Patentansprüche

1. Eine Darreichungsform, die in einer bestimmten Anzahl von Tageseinheiten für eine ununterbrochene, tägliche orale Verabreichung vorliegt, deren Wirkstoffkombination aus wenigstens einem Retinoid ausgewählt aus der Gruppe bestehend aus Acitretin [9-(4-Methoxy-2,3,6-trimettiyl-phenylr3,7-dimethyl-nona-2,4,6,8-tetraensäure], Etretinat [Ethyl 9-(4-methoxy-2,3,6-trimethyl-phenyl)-3,7-dimethyl-nona-2,4,6,8-tetraenat], Isotretinoin [3,7-Dimethyl-9-(2,6,6-trimethyl-1-cyclo-hexen-1-yl)-2,4,6,8-nonatetraensäure] und Tretinoin [3,7-Dimethyl-9-(2,6,6-trimethyl- 1-cyclohexenyl)nona-2,4,6,8-tetraensäure] und aus einer kontrazeptiv wirkenden Hormonkombination aus wenigstens einem Östrogen ausgewählt aus der Gruppe bestehend aus Ethinylestradiol und Östradiol als Östrogenkomponente und wenigstens einem Gestagen ausgewählt aus der Gruppe bestehend aus Chlormadinonacetat, 3β-Hydroxy-Chlormadinonacetat (17α-acetoxychloropregna-4,6-dien-3β-ol-20-on) und 3α-Hydroxy-Chlormadinonacetat (17α-acetoxy-chloropregna-4,6-dien-3α-ol-20-on) als Gestagenkomponente besteht, und die ggfs. mit einer bestimmten Anzahl von hormonfreien, nur das h Retinoid als Wirkstoff enthaltenen Tageseinheiten für eine daran anschließende, ununterbrochene, tägliche orale Verabreichung an Frauen kombiniert ist.

2. Eine Darreichungsform nach Anspruch 1, **dadurch gekennzeichnet, dass** als Retinoid Isotretinoin [3,7-Dimethyl-9-(2,6,6-trimethyl-1-cyclo-hexen-1-yl)-**2,4,6,8-nonatetraensäure] vorliegt.**

3. Eine Darreichungsform nach Anspruch 1 oder 2, **dadurch gekennzeichnet, dass** die Darreichungsform wenigstens 21 die Wirkstoffkombination enthaltenden Tageseinheiten und 7 bis 3 hormonfreie, nur das Retinoid als Wirkstoff enthaltenden Tageseinheiten aufweist.

4. Eine Darreichungsform nach einem der Ansprüche 1 bis 3, **dadurch gekennzeichnet, dass** die maximale Anzahl der Tageseinheiten der Darreichungsform einer ununterbrochenen, täglichen, oralen Verabreichung für 364 Tage entspricht, wobei 357 bis 361 die Wirkstoffkombination enthaltende Tageseinheiten zur ununterbrochenen, täglichen, oralen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen, oder die maximale Anzahl der Tageseinheiten der Darreichungsform einer ununterbrochenen, täglichen, oralen Verabreichung für 196 Tage entspricht, wobei 189 bis 193 die Wirkstoffkombination enthaltende Tageseinheiten zur ununterbrochenen, täglichen, oralen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen oder die maximale Anzahl der Tageseinheiten der Darreichungsform einer ununterbrochenen, täglichen, oralen Verabreichung für 168 Tage entspricht, wobei 161 bis 165 die Wirkstoffkombination enthaltende Tageseinheiten zur ununterbrochenen, täglichen, oralen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen.

5. Eine Darreichungsform nach einem der Ansprüche 1 bis 3, **dadurch gekennzeichnet, dass** die maximale Anzahl der Tageseinheiten der Darreichungsform einer ununterbrochenen, täglichen, oralen Verabreichung für 112 Tage entspricht, wobei 105 bis 109 die Wirkstoffkombination enthaltende Tageseinheiten zur ununterbrochenen, täglichen, oralen Verabreichung und 7 bis 3 hormonfreie, nur das Retinoid als Wirkstoff enthaltende Tageseinheiten zur anschließenden ununterbrochenen, täglichen, oralen Verabreichung an Frauen vorliegen oder die maximale Anzahl der Tageseinheiten der Darreichungsform einer ununterbrochenen, täglichen, oralen Verabreichung für 84 Tage entspricht, wobei 77 bis 81 die Wirkstoffkombination enthaltende Tageseinheiten zur ununterbrochenen, täglichen, oralen Verabreichung und 7 bis 3 hormonfreie, das Retinoid enthaltenden Tageseinheiten zur anschließenden ununterbrochenen, täglichen, oralen Verabreichung an Frauen vorliegen.

6. Eine Darreichungsform nach einem der Ansprüche 1-5, **dadurch gekennzeichnet, dass** die Darreichungsform aus maximal 13, zur ununterbrochenen, täglichen, oralen Verabreichung vorgesehenen Anordnungen aus jeweils 21 bis 25 die Wirkstoffkombination enthaltenden Tageseinheiten und jeweils 7 bis 3 hormonfreien, nur das Retinoid als Wirkstoff enthaltenden, zur daran anschließenden ununterbrochenen, täglichen, oralen Verabreichung an Frauen vorgesehenen Tageseinheiten besteht.

7. Eine Darreichungsform nach einem der Ansprüche 1 bis 6, **dadurch gekennzeichnet, dass** jede die hormonhaltige Wirkstoffkombination enthaltende Tageseinheit die Östrogenkomponente jeweils in einer den Menstruationszyklus stabilisierenden Menge und die Gestagenkomponente jeweils in einer kontrazeptiv wirkenden Menge enthält.

8. Eine Darreichungsform nach einem der Ansprüche 1 bis 7, **dadurch gekennzeichnet, dass** jede der die hormonhaltige Wirkstoffkombination enthaltenden Tageseinheiten jeweils dieselbe Menge der Gestagenkomponente und jeweils dieselbe Menge der Östrogenkomponente und jede Tageseinheit der Darreichungsform 1 bis 75 mg, vorzugsweise 3 bis 30 mg des Retinoids enthält.

9. Eine Darreichungsform nach Anspruch 8, **dadurch gekennzeichnet, dass** die zum Beginn der Verabreichung vorgesehenen 28 Tageseinheiten einer Darreichungsform jeweils mindestens 10 mg des Retinoids und die weiteren Tageseinheiten der Darreichungsform jeweils nur 1/2 bis 1/5 dieser Tages-Retinoidmenge enthalten.

10. Eine Darreichungsform nach Anspruch 8 oder 9, **dadurch gekennzeichnet, dass** die zum Beginn der Verabreichung vorgesehenen 28 Tageseinheiten einer Darreichungsform jeweils dieselbe Tages-Menge des Retinoids und die restlichen Tageseinheiten der Darreichungsform jeweils dieselbe, aber gegenüber den zum Beginn verabreichten 28 Tageseinheiten verminderte Tages-Menge des Retinoids aufweisen.

11. Verwendung von wenigstens einem Retinoid gemäß Anspruch 1 zur Herstellung einer Darreichungsform in Form von wenigstens 28 Tageseinheiten, wobei außer der Herstellung der letzten 7-3 Tageseinheiten bei der Herstellung der übrigen Tageseinheiten eine Hormonkombination gemäß einem der Ansprüche 1, 2, 7-10 mitverwendet wird, zur Behandlung und/oder Prophylaxe von Akne, Seborrhöe, Psoriasis und/oder Rosazea.

12. Verwendung nach Anspruch 11, zur Behandlung von *Acne aestivalis, Acne aggregata, Acne comedonica, Acne conglobata, Acne inversa, Acne nodularis, Acne papulopustulosa* und *Acne vulgaris,* vorzugsweise von *Acne vulgaris.*

## Claims

1. A dosage form which is present as a specific number of daily units for uninterrupted, daily oral administration, the active ingredient combination of which consists of at least one retinoid selected from the group consisting of acitretin [9-(4-methoxy-2,3,6-trimethylphenyl)-3,7-dimethylnona-2,4,6,8-tetraenoic acid], etretinate [ethyl 9-(4-methoxy-2,3,6-trimethylphenyl)-3,7-dimethylnona-2,4,6,8-tetraenoate], isotretinoin [3,7-dimethyl-9-(2,6,6-trimethyl-1-cyclohexen-1-yl)-2,4,6,8-nonatetraenoic acid] and tretinoin [3,7-dimethyl-9-(2,6,6-trimethyl-1-cyclohexenyl)nona-2,4,6,8-tetraenoic acid] and of a hormone combination with contraceptive action of at least one oestrogen component selected from the group consisting of ethinyl oestradiol and oestradiol and at least one gestagen selected from the group consisting of chlormadinone acetate, 3β-hydroxychlormadinone acetate (17α-acetoxychloropregna-4,6-dien-3β-ol-20-one) and 3α-hydroxychlormadinone acetate (17α-acetoxychloropregna-4,6-dien-3α-ol-20-one) as gestagen component, and which is optionally combined with a specific number of hormone-free daily units containing only the retinoid as active ingredient for immediately subsequent, uninterrupted, daily oral administration to women.

2. A dosage form according to claim 1, **characterised in that** isotretinoin [3,7-dimethyl-9-(2,6,6-trimethyl-1-cyclohexen-1-yl)-2,4,6,8-nonatetraenoic acid] is present as the retinoid.

3. A dosage form according to claim 1 or claim 2, **characterised in that** the dosage form comprises at least 21 daily units containing the active ingredient combination and 7 to 3 hormone-free daily units containing only the retinoid as active ingredient.

4. A dosage form according to any one of claims 1 to 3, **characterised in that** the maximum number of daily units of the dosage form corresponds to uninterrupted, daily, oral administration for 364 days, with 357 to 361 daily units containing the active ingredient combination for uninterrupted, daily, oral administration and 7 to 3 hormone-free daily units containing only the retinoid as active ingredient for subsequent uninterrupted, daily, oral administration to women, or the maximum number of daily units of the dosage form corresponds to uninterrupted, daily, oral administration for 196 days, with 189 to 193 daily units containing the active ingredient combination for uninterrupted, daily, oral administration and 7 to 3 hormone-free daily units containing only the retinoid as active ingredient for subsequent uninterrupted, daily, oral administration to women, or the maximum number of daily units of the dosage form corresponds to uninterrupted, daily, oral administration for 168 days, with 161 to 165 daily units containing the active ingredient combination for uninterrupted, daily, oral administration and 7 to 3 hormone-free daily units containing only the retinoid as active ingredient for subsequent uninterrupted, daily, oral administration to women.

5. A dosage form according to any one of claims 1 to 3, **characterised in that** the maximum number of daily units of the dosage form corresponds to uninterrupted, daily, oral administration for 112 days, with 105 to 109 daily units containing the active ingredient combination for uninterrupted, daily, oral administration and 7 to 3 hormone-free daily units containing only the retinoid as active ingredient for subsequent uninterrupted, daily, oral administration to women or the maximum number of daily units of the dosage form corresponds to uninterrupted, daily, oral administration for 84 days, with 77 to 81 daily units containing the active ingredient combination for uninterrupted, daily, oral administration and 7 to 3 hormone-free daily units containing the retinoid for subsequent uninterrupted, daily, oral administration to women.

6. A dosage form according to any one of claims 1-5, **characterised in that** the dosage form consists of at most 13 arrangements provided for uninterrupted, daily, oral administration, said arrangements comprising in each case 21 to 25 daily units containing the active ingredient combination and in each case 7 to 3 hormone-free daily units containing only the retinoid as active ingredient provided for immediately subsequent uninterrupted, daily, oral administration to women.

7. A dosage form according to any one of claims 1 to 6, **characterised in that** each daily unit containing the hormone-containing active ingredient combination contains the oestrogen component in each case in a quantity which stabilises the menstrual cycle and the gestagen component in each case in a quantity with a contraceptive action.

8. A dosage form according to any one of claims 1 to 7, **characterised in that** each of the daily units containing the hormone-containing active ingredient combination contains in each case the same quantity of the gestagen component and in each case the same quantity of the oestrogen component and each daily unit of the dosage form contains 1 to 75 mg, preferably 3 to 30 mg of the retinoid.

9. A dosage form according to claim 8, **characterised in that** each of the 28 daily units of a dosage form provided for the starting period of administration contains at least 10 mg of the retinoid and each of the further daily units of the dosage form contains only 1/2 to 1/5 of this daily quantity of retinoid.

10. A dosage form according to claim 8 or claim 9, **characterised in that** each of the 28 daily units of a dosage form provided for the starting period of administration comprises the same daily quantity of the retinoid and each of the remaining daily units of the dosage form comprises the same daily quantity of the retinoid, which is, however, reduced relative to the 28 daily units administered at the start of administration.

11. Use of at least one retinoid according to claim 1 for producing a dosage form in the form of at least 28 daily units in combination with a hormone combination according to any one of claims 1, 2, 7-10 with the exception of the production of the final 7-3 daily units, for treating and/or preventing acne, seborrhoea, psoriasis and/or rosacea.

12. Use according to claim 11, for treating *acne aestivalis, acne aggregata, acne comedonica, acne conglobata, acne inversa, acne nodularis, acne papulopustulosa* and *acne vulgaris,* preferably *acne vulgaris.*

## Revendications

1. Forme pharmaceutique, qui se présente en un nombre déterminé d'unités journalières pour une administration orale journalière ininterrompue, dont la combinaison d'agents actifs est constituée d'au moins un rétinoïde choisi dans le groupe constitué par l'acitrétine [acide 9-(4-méthoxy-2,3,6-triméthyl-phényl)-3,7-diméthyl-nona-2,4,6,8-tétraénoïque], l'étrétinate [9-(4-méthoxy-2,3,6-triméthyl-phényl)-3,7-diméthyl-nona-2,4,6,8-tétraénate d'éthyle], l'isotrétinoïne [acide 3,7-diméthyl-9-(2,6,6-triméthyl-1-cyclo-hexén-1-yl)-2,4,6,8-nonatétraénoïque] et la trétinoïne [acide 3,7-diméthyl-9-(2,6,6-triméthyl-1-cyclohexényl)nona-2,4,6,8-tétraénoïque] et d'une combinaison d'hormones à action contraceptive constituée d'au moins un oestrogène choisi dans le groupe constitué par l'éthinylestradiol et l'estradiol en tant que composant oestrogène et d'au moins un gestagène choisi dans le groupe constitué par l'acétate de chlormadinone, l'acétate de 3β-hydroxychlormadinone (17α-acétoxy-chloroprégna-4,6-dièn-3β-ol-20-one) et l'acétate de 3α-hydroxy-chlormadinone (17α-acétoxy-chloroprégna-4,6-dièn-3α-ol-20-one) en tant que composant gestagène, et qui est éventuellement combinée avec un nombre déterminé d'unités journalières sans hormones, ne contenant que le rétinoïde en tant qu'agent actif, pour une administration orale journalière ininterrompue ultérieure chez les femmes.

2. Forme pharmaceutique selon la revendication 1, **caractérisée en ce que** l'isotrétinoïne [acide 3,7-diméthyl-9-(2,6,6-triméthyl-1-cyclo-hexén-1-yl)-2,4,6,8-nonatétraénoïque] est présente en tant que rétinoïde.

3. Forme pharmaceutique selon la revendication 1 ou 2, **caractérisée en ce que** la forme pharmaceutique comprend au moins 21 unités journalières contenant la combinaison d'agents actifs et 7 à 3 unités journalières sans hormones, ne contenant que le rétinoïde en tant qu'agent actif.

4. Forme pharmaceutique selon l'une quelconque des revendications 1 à 3, **caractérisée en ce que** le nombre maximal des unités journalières de la forme pharmaceutique correspond à une administration orale journalière ininterrompue pendant 364 jours, 357 à 361 unités journalières contenant la combinaison d'agents actifs pour l'administration orale journalière ininterrompue et 7 à 3 unités journalières sans hormones, ne contenant que le rétinoïde en tant qu'agent actif, pour l'administration orale journalière ininterrompue ultérieure chez les femmes, étant présentes, ou le nombre maximal des unités journalières de la forme pharmaceutique correspond à une administration orale journalière ininterrompue pendant 196 jours, 189 à 193 unités journalières contenant la combinaison d'agents actifs pour l'administration orale journalière ininterrompue et 7 à 3 unités journalières sans hormones, ne contenant que le rétinoïde en tant qu'agent actif, pour l'administration orale journalière ininterrompue ultérieure chez les femmes, étant présentes, ou le nombre maximal des unités journalières de la forme pharmaceutique correspond à une administration orale journalière ininterrompue pendant 168 jours, 161 à 165 unités journalières contenant la combinaison d'agents actifs pour l'administration orale journalière ininterrompue et 7 à 3 unités journalières sans hormones, ne contenant que le rétinoïde en tant qu'agent actif, pour l'administration orale journalière ininterrompue ultérieure chez les femmes, étant présentes.

5. Forme pharmaceutique selon l'une quelconque des revendications 1 à 3, **caractérisée en ce que** le nombre maximal des unités journalières de la forme pharmaceutique correspond à une administration orale journalière ininterrompue pendant 112 jours, 105 à 109 unités journalières contenant la combinaison d'agents actifs pour l'administration orale journalière ininterrompue et 7 à 3 unités journalières sans hormones, ne contenant que le rétinoïde en tant qu'agent actif, pour l'administration orale journalière ininterrompue ultérieure chez les femmes, étant présentes, ou le nombre maximal des unités journalières de la forme pharmaceutique correspond à une administration orale journalière ininterrompue pendant 84 jours, 77 à 81 unités journalières contenant la combinaison d'agents actifs pour l'administration orale journalière ininterrompue et 7 à 3 unités journalières sans hormones, contenant le rétinoïde, pour l'administration orale journalière ininterrompue ultérieure chez les femmes, étant présentes.

6. Forme pharmaceutique selon l'une quelconque des revendications 1 à 5, **caractérisée en ce que** la forme pharmaceutique est constituée d'au plus 13 agencements prévus pour l'administration orale journalière ininterrompue, constitués respectivement de 21 à 25 unités journalières contenant la combinaison d'agents actifs et respectivement de 7 à 3 unités journalières sans hormones, ne contenant que le rétinoïde en tant qu'agent actif, pour l'administration orale journalière ininterrompue ultérieure chez les femmes.

7. Forme pharmaceutique selon l'une quelconque des revendications 1 à 6, **caractérisée en ce que** chaque unité journalière contenant la combinaison d'agents actifs contenant des hormones contient le composant oestrogène respectivement en une quantité stabilisant le cycle menstruel et le composant gestagène respectivement en une quantité à action contraceptive.

8. Forme pharmaceutique selon l'une quelconque des revendications 1 à 7, **caractérisée en ce que** chacune des unités journalières contenant la combinaison d'agents actifs contenant des hormones contient à chaque fois la même quantité du composant gestagène et à chaque fois la même quantité du composant oestrogène, et chaque unité journalière de la forme pharmaceutique contient 1 à 75 mg, de préférence 3 à 30 mg, du rétinoïde.

9. Forme pharmaceutique selon la revendication 8, **caractérisée en ce que** les 28 unités journalières d'une forme pharmaceutique prévues pour le début de l'administration contiennent respectivement au moins 10 mg du rétinoïde et les unités journalières ultérieures de la forme pharmaceutique ne contiennent respectivement que 1/2 à 1/5 de cette quantité de rétinoïde journalière.

10. Forme pharmaceutique selon la revendication 8 ou 9, **caractérisée en ce que** les 28 unités journalières d'une forme pharmaceutique prévues pour le début de l'administration contiennent à chaque fois la même quantité journalière du rétinoïde et les unités journalières restantes de la forme pharmaceutique contiennent à chaque fois la même quantité journalière du rétinoïde, toutefois réduite par rapport aux 28 unités journalières administrées au début.

11. Utilisation d'au moins un rétinoïde selon la revendication 1 pour la fabrication d'une forme pharmaceutique sous la forme d'au moins 28 unités journalières, outre la fabrication des 7 à 3 dernières unités journalières, une combinaison d'hormones selon l'une quelconque des revendications 1, 2, 7 à 10 étant utilisée lors de la fabrication des autres unités journalières, pour le traitement et/ou la prophylaxie de l'acné, de la séborrhée, du psoriasis et/ou de l'acné rosacée.

12. Utilisation selon la revendication 11, pour le traitement d'*Acne aestivalis, Acne aggregata, Acne comedonica, Acne conglobata, Acne inversa, Acne nodularis, Acne papulopustulosa* et *Acne vulgaris,* de préférence d'*Acne vulgaris.*
